# EUROPEAN PATENT APPLICATION

(11) **EP 4 623 875 A1**
(43) Date of publication of application: **01.10.2025**
(21) Application number: 24167792.1
(22) Date of filing: 28.03.2024
(51) Int. Cl.: A61F 9/00, B65D 47/18, B65D 41/00

(54) **DEVICE FOR DISPENSING A LIQUID**

(71) Applicant: Röchling Medical SE, 68165 Mannheim (DE)
(72) Inventor: Wolf, Tobias, 07338 Kaulsdorf (DE); Tröbs, Ralf, 07333 Unterwellenborn (DE)
(74) Representative: Ullrich & Naumann PartG mbB

(57) **Abstract**

The invention relates to a device for dispensing a liquid, preferably an aqueous solution, comprising a vessel for containing the liquid, a dispensing dropper with a dispensing tip and a cap, wherein the cap and the vessel comprise corresponding fixation elements, wherein the cap comprises a sealing ring and that a lateral wall of the sealing ring is in engagement with a lateral surface of the dispensing tip when the vessel is closed by the cap.

## Description

The invention relates to a device for dispensing a liquid, preferably an aqueous solution, comprising a vessel for containing the liquid, a dispensing dropper with a dispensing tip and a cap, wherein the cap and the vessel comprise corresponding fixation elements.

Although applicable to any kind of liquids, the present invention will be described with regard to ophthalmic liquids, for example ophthalmic drugs or contact lens solutions.

Known devices for dispensing an ophthalmic liquid comprise a vessel for the liquid, a dispensing dropper and a cap. By squeezing the vessel, a single drop of the liquid can be dispensed through the dispensing tip for applying the liquid to an eye. To protect the contents of the vessel from contamination, which is particularly important if the device is a pharmaceutical primary packaging, it has to be securely closed with the cap. Further, the cap serves to prevent liquid from leaking or diffusing out of the vessel.

EP 2 111 362 B1 shows an assembly for dispensing a medical liquid. The assembly comprises a vessel for containing a liquid, a dispensing dropper being connected to the vessel and a cap for closing the vessel. The cap can be attached to the vessel via a screw connection. Further, the cap comprises a nub, which is pressed onto a distal opening of the dispensing dropper when the cap is screwed onto the vessel. Hence, the nub serves to seal the inside of the vessel against contaminants and prevents leakage of the liquid.

One of the disadvantages however is that the cap has to be screwed with a relatively high torque onto the vessel to ensure that the nub is pressed hard enough onto the opening of the dispensing dropper. However, if the torque is too high, the cap damages the distal opening of the dispensing dropper that comprises the droplet-generating structure. If this structure is damaged the liquid is not dispensed as a droplet but as a jet. However, especially when dispensing a medical fluid, it is essential that it is dispensed in the required quantity and form. Further, dispensing an ophthalmic liquid in form of a jet instead of a drop may lead to an injury of the eye which has to be prevented.

Further devices for dispensing eye drops are disclosed in US 7 178 703 B2 and EP 2 531 154 B1.

One of the objectives of the present invention is therefore to provide a device for dispensing a liquid, preferably an aqueous solution, which is easy to handle and provides a reliable protection against liquid leakage and protects the contents of the vessel from contaminants.

A further objective of the present invention is to provide an alternative device for dispensing a liquid.

In an embodiment, the present invention may solve at least one of the objectives by a device for dispensing a liquid, preferably an aqueous solution, comprising a vessel for containing the liquid, a dispensing dropper with a dispensing tip and a cap, wherein the cap and the vessel comprise corresponding fixation elements,
characterized in that the cap comprises a sealing ring and that a lateral wall of the sealing ring is in engagement with a lateral surface of the dispensing tip when the vessel is closed by the cap.

One of the advantages may be that the, preferably inner, lateral wall of the sealing ring seals laterally at the dispensing tip, whereby a height-independent sealing function is realized once the lateral wall of the sealing ring and the lateral surface of the dispensing tip are engaged. Preferably the inner lateral wall of the sealing ring may enclose the outer laterally sealing surface of the dispensing tip. Further, damage to the dispensing tip of the dispensing dropper may be prevented since it is not necessary to attach the cap tightly to the vessel because of the lateral sealing function of the device. Also, neither the sealing ring nor any other part of the cap may be in contact with the distal opening of the distal tip. Hence, the dispensing opening cannot be damaged by the cap. Furthermore, an advantage of the device may be that it could be used as a pharmaceutical primary packaging, i.e. a packaging that is in direct contact with a pharmaceutical liquid, for example an ophthalmological drug. Moreover, the liquid being stored in the vessel could be an aqueous solution, for example an ophthalmological drug or a contact lens care product.

Further features, advantages and preferred embodiments are disclosed or may become apparent in the following.

According to a preferred embodiment of the invention, said vessel is designed to store liquid in the range of 2,5 ml and 50 ml, preferably 5 ml and 20 ml, preferably 5 ml and 15 ml. This may have the advantage that the device may be used as a primary packaging for ophthalmic solutions and/or for contact lens solutions.

According to a further preferred embodiment of the invention, said fixation elements of the cap and of the vessel are corresponding threads. This may allow the cap to be screwed onto the vessel such that the lateral wall of the sealing ring and the lateral surface of the dispensing tip contact each other and seal the inside of the vessel.

According to a further preferred embodiment of the invention, said vessel and said cap comprise corresponding end stop elements and wherein the cap can only be screwed onto the vessel until the end stop elements engage with each other. The end stop elements may be arranged separate from the sealing wall and from the sealing surface. The end stop elements may provide the advantage of a mechanical end stop so that damage to the dispensing tip, e.g. a drop-forming distal end of the distal tip, caused by tightening the screw connection too much may be prevented.

According to a further preferred embodiment of the invention, said lateral wall of the sealing ring extends at least 2,0 mm, preferably at least 2,1 mm from a basis body of the cap, and/or wherein the lateral wall of the sealing rings extends less than 2,3 mm from a basis body of the cap. A lateral wall extending at least 2,0 mm from a basis body may have the advantage that the surface area of the lateral wall is large enough to achieve sufficient sealing, whereas a lateral wall extending at least 2,1 mm from the basis body provides an even better sealing. Further, a lateral wall extending less than 2,3 mm from the basis body may have the advantage that the sealing ring is a small as possible but achieves a reliable sealing effect. A sealing wall extending 2,1 mm from the basis body may provide an ideal sealing effect.

According to a further preferred embodiment of the invention, said lateral surface of the dispensing tip extends at least 2,0 mm, preferably at least 2,3 mm, more preferably at least 1 mm, into a longitudinal direction of the dispensing dropper, and/or wherein the lateral surface of the dispensing tip extends less than 2,5 mm into a longitudinal direction of the dispensing dropper. A lateral surface extending at least 2,0 mm into a longitudinal direction of the dispensing dropper may have the advantage that the surface area of the lateral surface is large enough to achieve sufficient sealing, whereas a lateral surface extending at least 2,3 mm into a longitudinal direction of the dispensing dropper provides an even better sealing. Further, a lateral surface extending less than 2,5 mm into a longitudinal direction of the dispensing dropper may have the advantage that the sealing surface is as small as possible but achieves a reliable sealing effect. A sealing surface extending 2,3 mm into a longitudinal direction of the dispensing dropper may provide an ideal sealing effect.

According to a further preferred embodiment of the invention, said dispensing dropper is form-fitted and/or force-fitted to the vessel and/or wherein a distal opening of the dispensing tip comprises a, preferably surrounding, chamfer. One of the advantages may be that the dispensing dropper is securely connected to the vessel. Further, the dispensing dropper may be prevented from falling off the vessel.

According to a further preferred embodiment of the invention, said dispensing dropper and said vessel each comprise an undercut, preferably a snap ring, such that the dispensing dropper can be fixed to the vessel. This may ensure that the assembly of vessel and tip can be carried out automatically by simply pressing these components together.

According to a further preferred embodiment of the invention, a tamper-evident ring is connected to the cap and/or to the vessel, preferably form-fitted and/or force-fitted, and/or wherein a tamper-evident ring is integrally formed with the cap and/or with the vessel. A tamper-evident ring may provide the advantage that a user can easily recognize if the cap was already detached from the vessel. This may be particularly advantageous for medical liquids in order to ensure safe use of the medication.

According to a further preferred embodiment of the invention, said vessel comprises a centering element, preferably a centering ring, and wherein the centering element is arranged between the thread and a bottom of the vessel. This may ensure that the cap is centered in relation to the bottle when it is attached to the vessel.

According to a further preferred embodiment of the invention, said dispensing dropper and said vessel comprise corresponding lateral sealing surfaces and wherein a lateral sealing surface of the vessel is in engagement with a corresponding lateral sealing surface of the dispensing dropper. This may create a tight seal between the dispensing dropper and the vessel. Hence, diffusion of the liquid out of the bottle may be prevented.

According to a further preferred embodiment of the invention, an opening torque for unscrewing the cap from the vessel is in the range of 25 N·cm to 70 N·cm. This feature may allow for easier unscrewing the cap from the vessel by hand. Additionally or alternatively, the cap may comprise a corrugation, preferably with a minimum of 3 ribs and a maximum of 25 ribs, more preferably wherein each rib extending at least 0,3 mm and at most 2 mm from a cap body. This may enhance the gripping of the cap by hand.

According to a further preferred embodiment of the invention, said cap comprises a high-density polyethylene, HDPE, and/or wherein said dispensing dropper comprises a low density polyethylene, LDPE, and/or wherein said vessel comprises a low density polyethylene, LDPE, and/or wherein a tamper-evident ring comprises a low density polyethylene, LDPE. These materials may have the advantages that they can be sterilized and are easy to process, e.g. in injection moulding.

According to a further preferred embodiment of the invention, said vessel comprises a squeezing portion and wherein the vessel is elastic against a force being applied to the squeezing portion of up to 70 N, preferably of up to 60 N. This may have the advantage that drops can also be dispensed by impaired persons by squeezing the vessel.

According to a further preferred embodiment of the invention, a mounting force for connecting the dispensing dropper to the vessel is less than 100 N, preferably less than 90 N, and/or wherein the mounting force for connecting the dispensing dropper to the vessel is greater than 50 N. This may ensure that the mounting force is less than the upset resistance of the vessel so that deformation of the vessel is avoided and also high enough that the dispensing dropper can be securely attached to the vessel. Alternatively or additionally a distal opening of the dispensing tip can comprise a diameter in the range from 0,3 mm to 2,5 mm, preferably from 0,8 mm to 2,3 mm. This may ensure that drops of a reproducibly size, preferably eye drops, are dispensed.

According to a further preferred embodiment of the invention, a distal opening of the dispensing tip comprises a, preferably surrounding, chamfer. One of the advantages may be that drops detach easily from the dispensing tip.

According to a further preferred embodiment of the invention, at least two, preferably at least three, more preferably four, centering ribs are arranged at the distal tip of the dispensing dropper, preferably wherein the centering ribs are arranged equidistantly to each other when viewed in a circumferential direction. This may provide that the cap is automatically centered when it is attached to the vessel.

There are several ways how to design and further develop the teaching of the present invention in an advantageous way. To this end, it is to be referred to the patent claims subordinate to patent claim 1 on the one hand and to the following explanation of preferred examples of embodiments of the invention, illustrated by the drawing on the other hand. In connection with the explanation of the preferred embodiments of the invention by the aid of the drawing, generally preferred embodiments and further developments of the teaching will be explained.

In the drawings
- Fig. 1: shows a device for dispensing a liquid according to an embodiment of the invention in an exploded view;
- Fig. 2: shows a cross-section of the device for dispensing a liquid of Figure 1;
- Fig. 3: shows a cross-section of the vessel of the device for dispensing a liquid of Figure 1;
- Fig. 4: shows the vessel of Figure 3 in a side view;
- Fig. 5: shows a cross-section of the cap of the device for dispensing a liquid of Figure 1;
- Fig. 6: shows a cross-section of the cap of Figure 5;
- Fig. 7: shows the cap of Figure 5 in a side view;
- Fig. 8: shows a cross-section of the tamper-evident ring of the device for dispensing a liquid of Figure 1;
- Fig. 9: shows a further cross-section of the tamper-evident ring of Figure 8;
- Fig. 10: shows the dispensing dropper of the device for dispensing a liquid of Figure 1 in a perspective view;
- Fig. 11: shows a cross-section of the dispensing dropper of Figure 10:
- Fig. 12: shows a further cross-section of the dispensing dropper of Figure 10:
- Fig. 13: shows a cross-section of a detail of the cap and of the vessel of Figure 1;
- Fig. 14: shows a cross-section of a detail of the dispensing dropper and of the vessel of Figure 1;
- Fig. 15: shows a cross-section of a detail of the cap and of the dispensing dropper of Figure 1;
- Fig. 16: shows a further cross-section of a detail of the cap and of the dispensing dropper of Figure 1;
- Fig. 17: shows a cross-section of a detail of the tamper-evident ring and of the vessel of Figure 1;
- Fig. 18: shows a cross-section of a detail of the tamper-evident ring and of the cap of Figure 1;

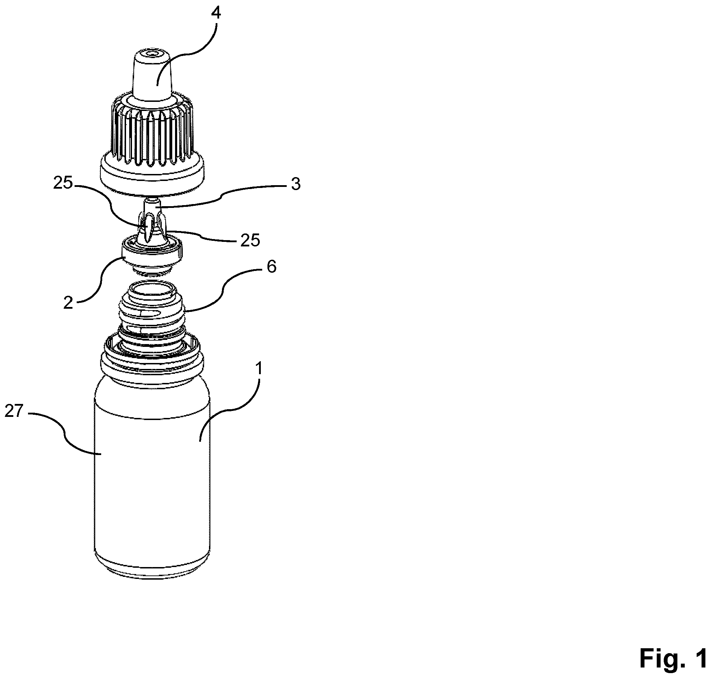

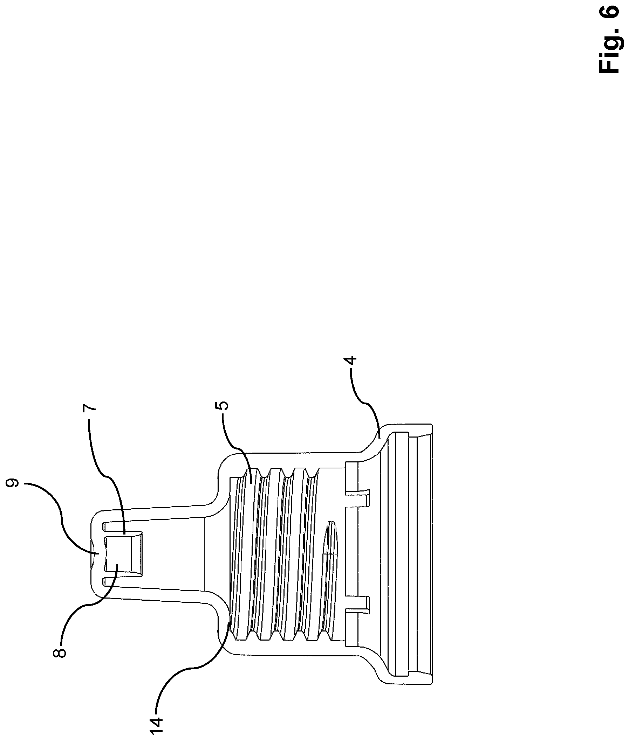

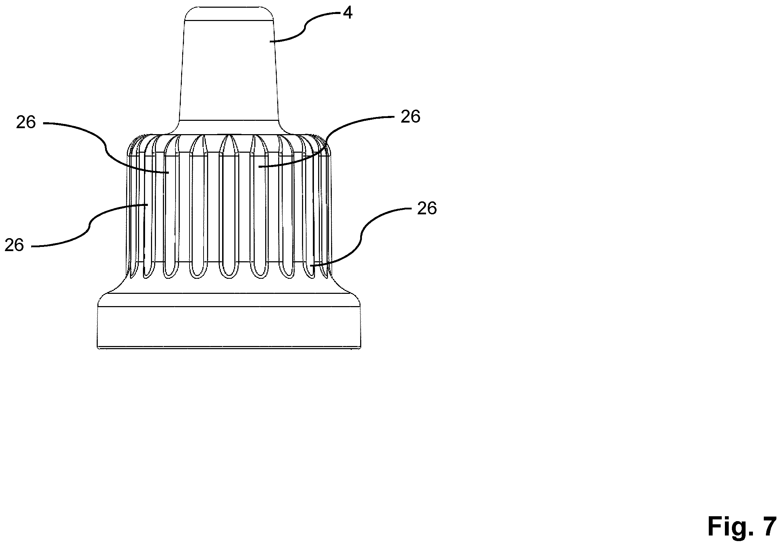

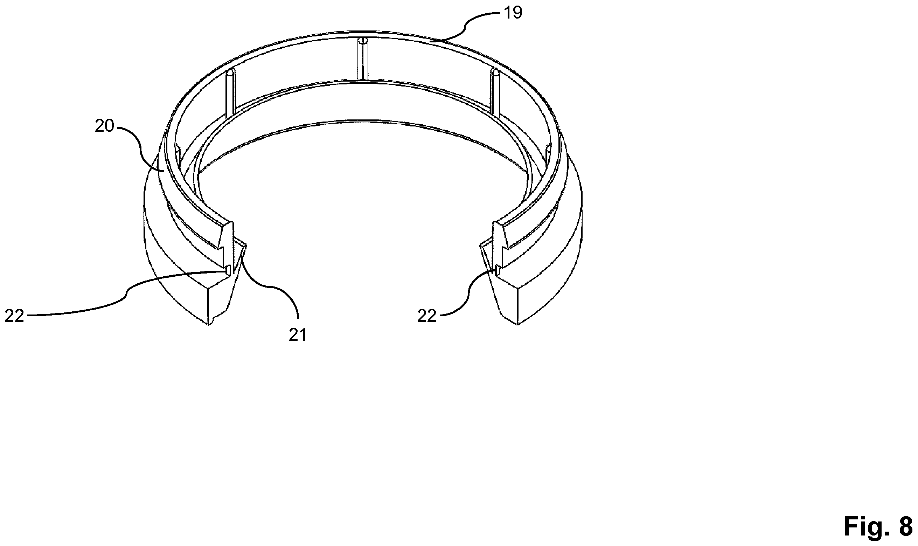

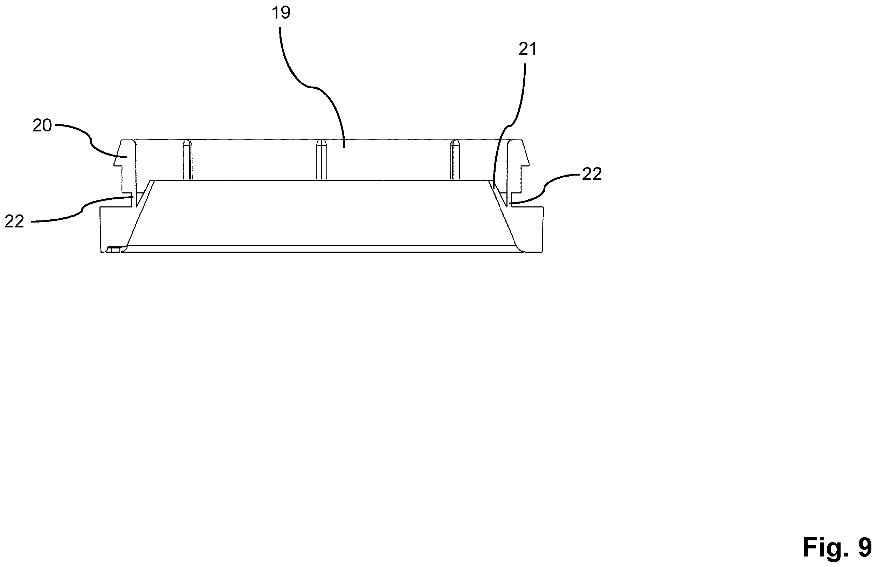

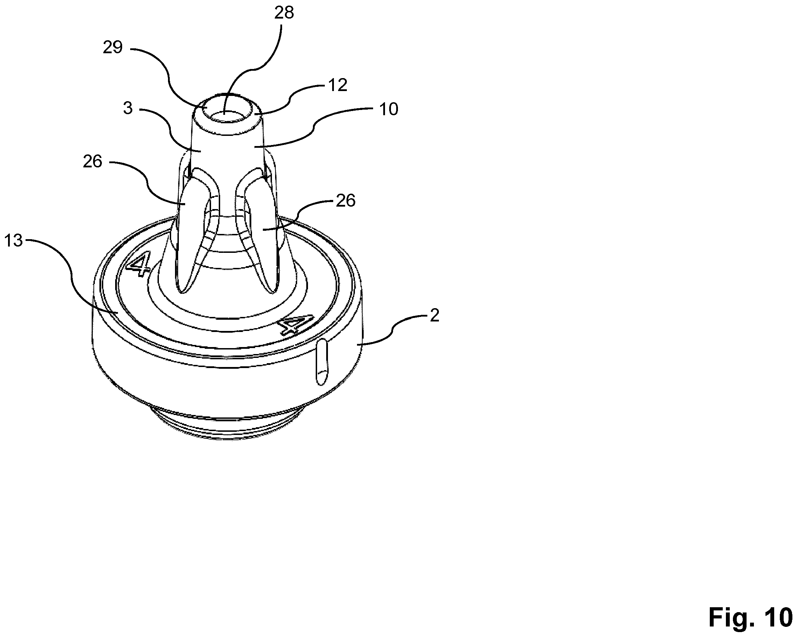

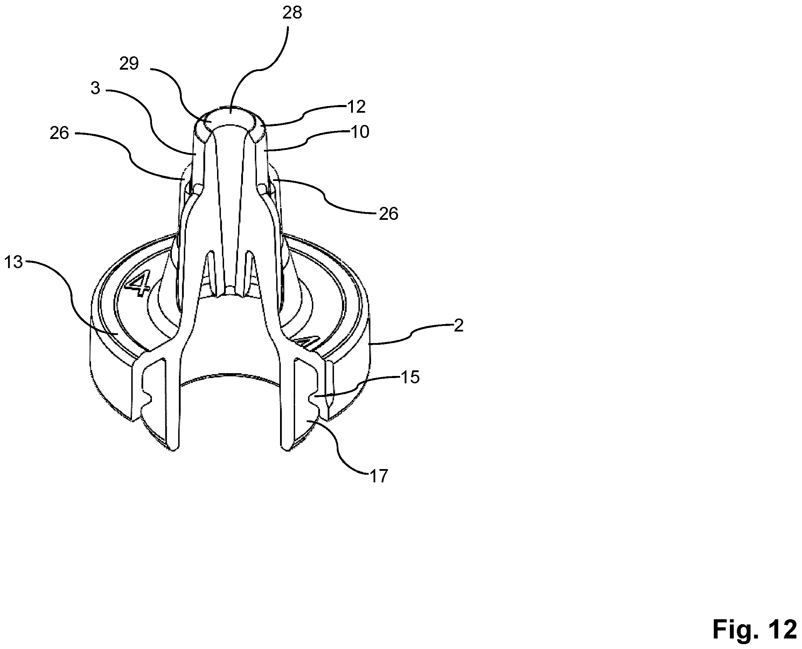

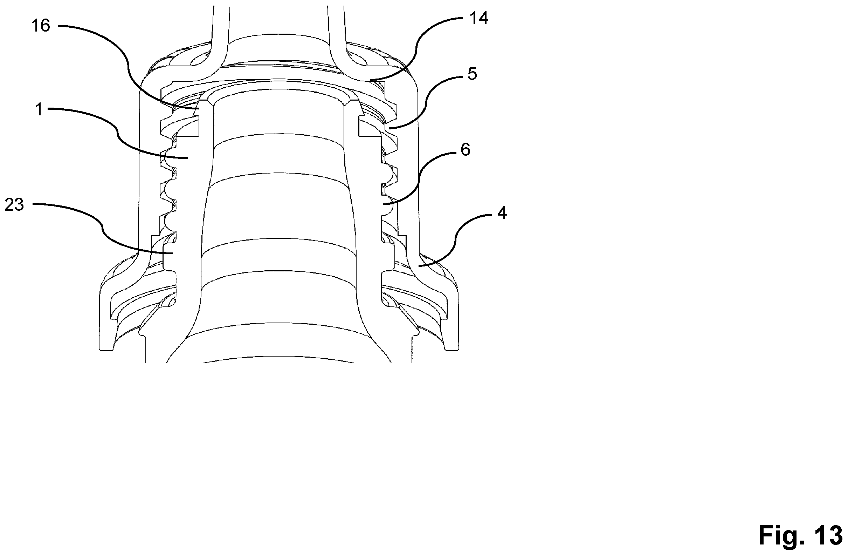

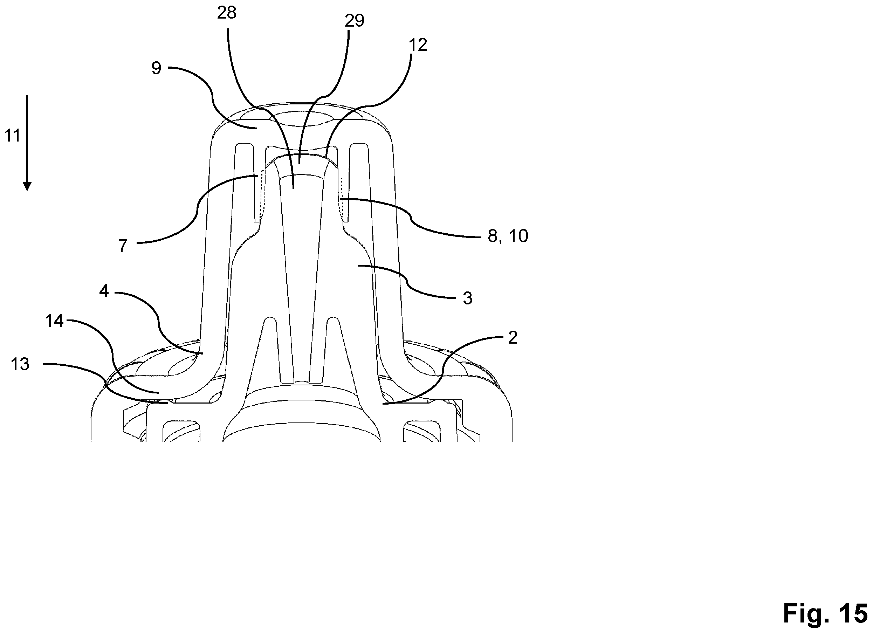

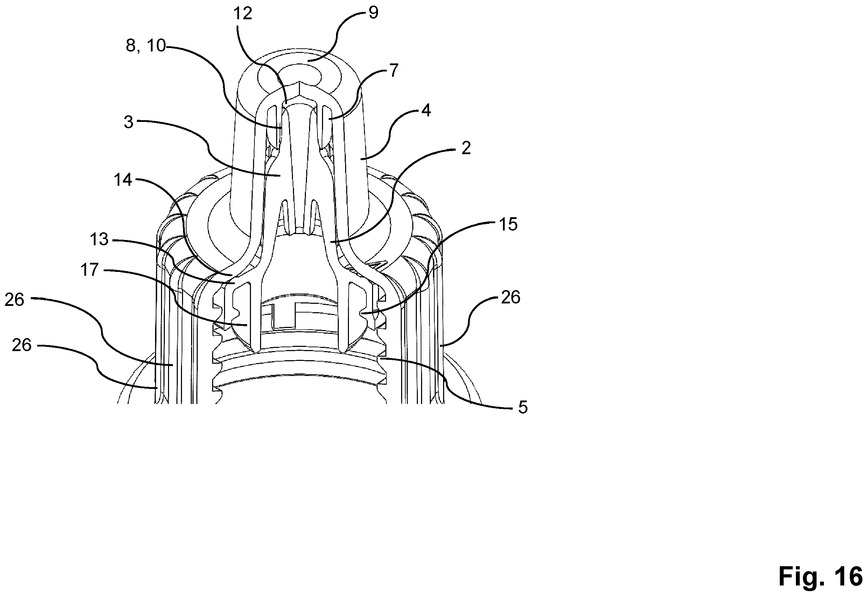

Figures 1 to 18 show a device for dispensing a liquid according to an embodiment of the invention in different views.

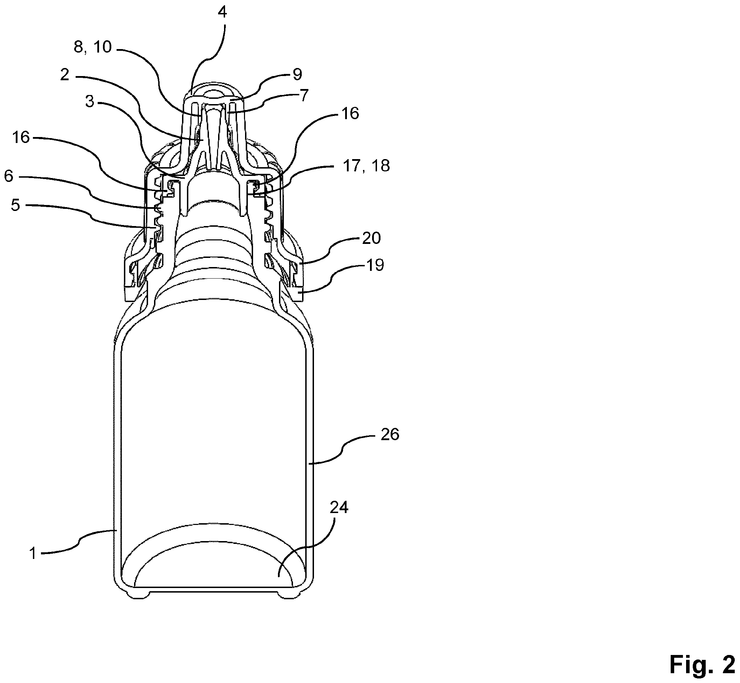

As can be seen in Figures 1 and 2 the device for dispensing a liquid comprises a vessel 1 for containing the liquid, a dispensing dropper 2 with a dispensing tip 3 and a cap 4. The cap 4 and the vessel 1 comprise corresponding fixation elements 5, 6 in the form of threads. Hence, the cap 4 can be connected to the vessel 1 via a screw connection.

Figures 2, 5, 6, 15 and 16 show that the cap 4 comprises a sealing ring 7. The lateral wall 8 of the sealing ring 7 extends from a basis body 9 of the cap and is in engagement with the lateral surface 10 of the dispensing tip 3 when the vessel 1 is closed by the cap 4. The lateral surface 10 of the dispensing tip 3 extends into a longitudinal direction 11 of the dispensing dropper 2. Hence, the inner lateral wall 8 of the sealing ring 7 and the outer lateral surface 10 of the dispensing tip 3 realize a height-independent sealing function. Further, since the cap 4 is not in contact with the distal end 12 of the dispensing tip 3, when the cap 4 is attached to the dispensing tip 3, damage to the dispensing tip 4 is prevented.

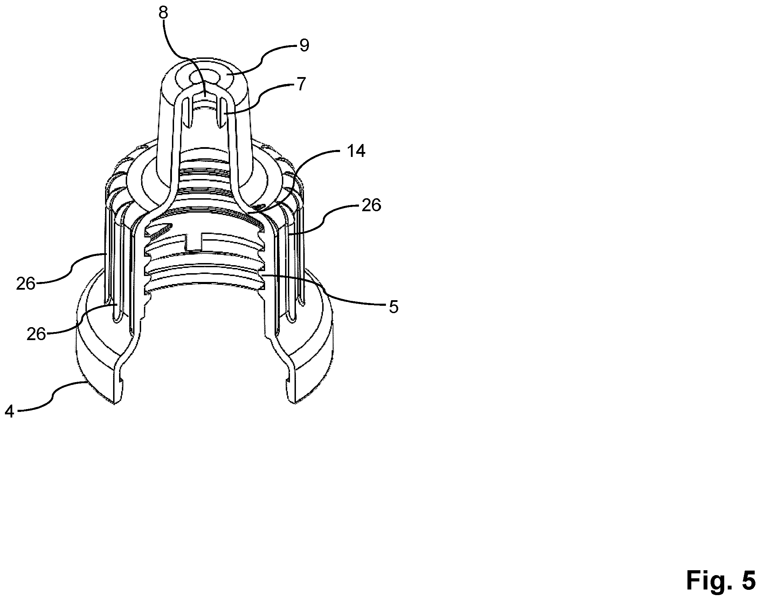

Moreover, the vessel 1 and the cap 4 comprise corresponding end stop elements 13, 14 such that the cap 4 can only be screwed onto the vessel 1 until the end stop elements 13, 14 engage with each other, as can be seen in Figures 5, 6, 10, 11, 12, 13, 14, 15, and 16.

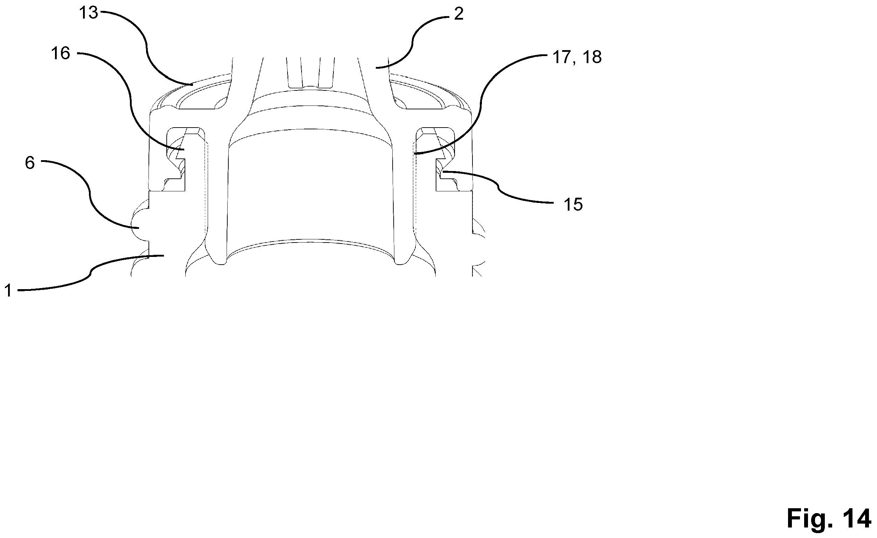

Figures 2 and 14 show that the dispensing dropper 2 and the vessel 1 each comprise an undercut 15, 16 in the form of a snap ring such that the dispensing dropper 2 can be fixed to the vessel 1. In addition, the dispensing dropper 2 and the vessel 1 comprise corresponding lateral sealing surfaces 17, 18 that are in engagement with each other. Therefore, the dispensing dropper 2 and the vessel 1 are tightly connected to each other for securely preventing leakage of the content of the vessel 1.

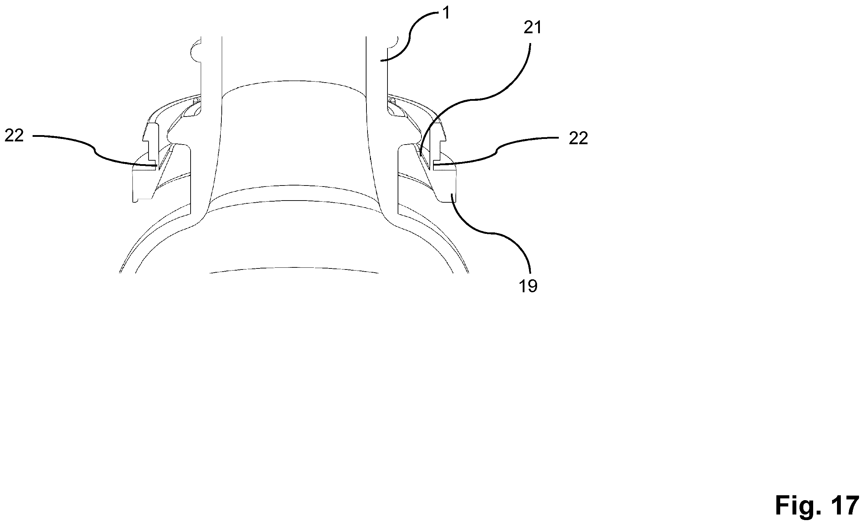

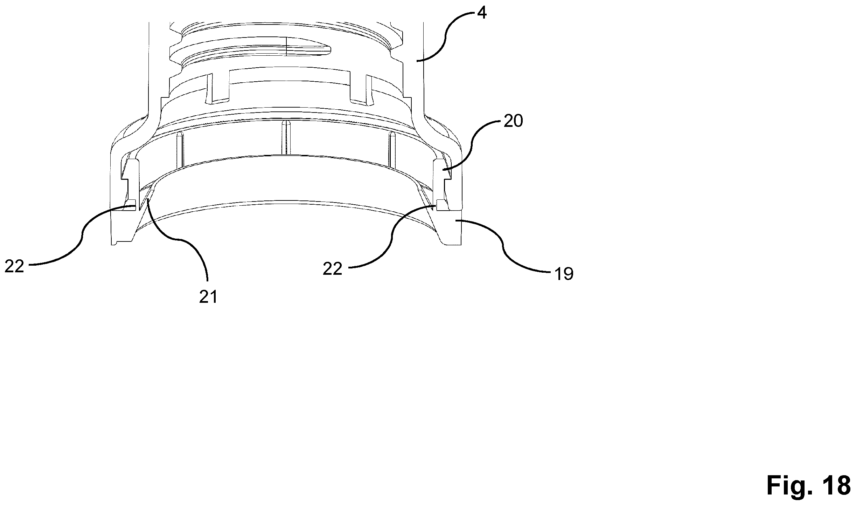

Figures 2, 8 and 9 show a tamper-evident ring 19. The tamper-evident ring 19 is connected to the vessel 1 and to the cap 4 respectively via a snap ring 20, 21 (Figures 17 and 18). Further, the tamper-evident ring 19 comprises tamper-evident connecting elements 22, wherein the tamper-evident connecting elements 22 are configured such to break off when the cap 4 is unscrewed from the vessel 1 for the first time. Hence, the tamper-evident connecting elements 22 indicate the originality of the device according to an embodiment of the invention.

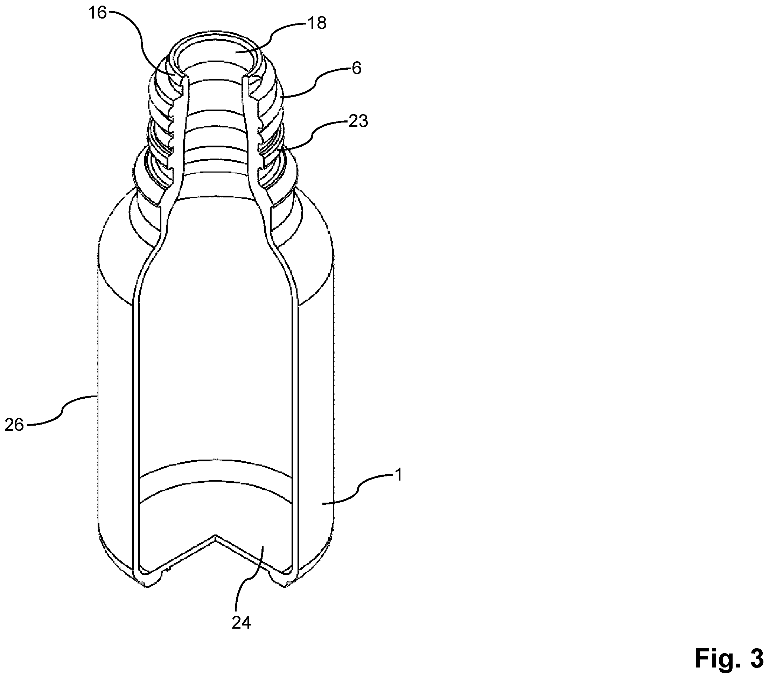

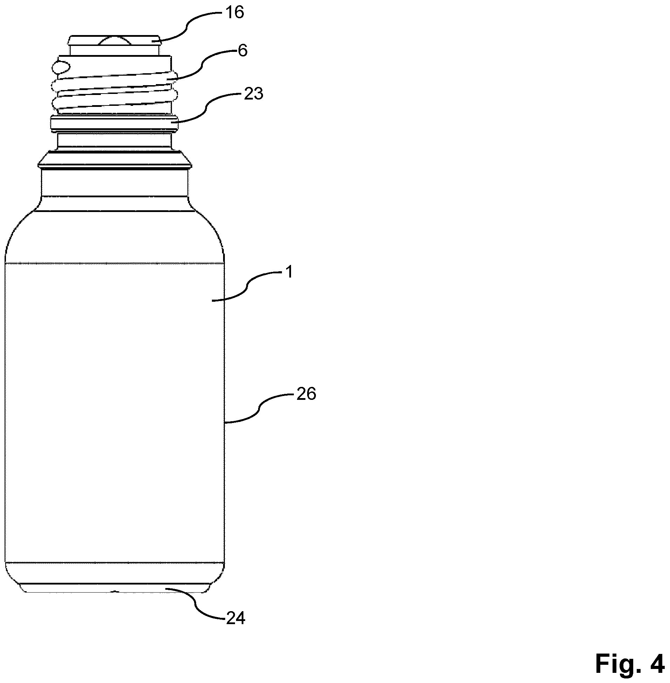

Furthermore, the vessel 1 comprises a centering ring 23 that is arranged between the fixation element 6 and the bottom 24 of the vessel 1 (Figures 3 and 4). The centering ring 23 serves to center the cap 4 when it is connected to the vessel 1. Further, the dispensing dropper 2 comprises centering ribs 25. In this embodiment the centering ribs 25 are arranged equidistantly to each other when viewed in a circumferential direction. When attaching the cap 1 to the vessel the centering ribs 25 align the cap 1 so that the threads of the vessel 1 and the cap 4 interlock properly.

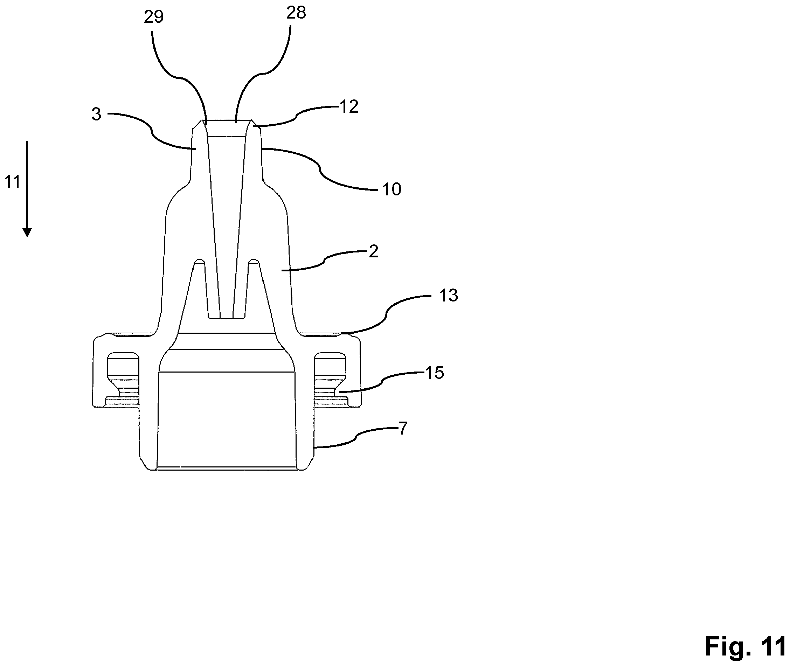

The device shown in Figures 1 to 18 can be used as a medical primary packaging, for example to dispensing an ophthalmic liquid. For dispensing the liquid, the cap 4 must be unscrewed from the vessel 1 which is facilitated by the corrugations 26 being arranged at the cap 4 for enhancing the user's grip (Figures 5, 7 and 16). When the cap 4 is detached from the vessel 1, the user squeezes the vessel 1 at the squeezing portion 27. The liquid is thus dispensed drop by drop from the distal opening 28 of the dispensing dropper 2. Figure 11 shows that the distal opening 28 has a chamfer 29 for dispensing the single drops.

At least one embodiment may provide at least one of the following features and/or at least one of the following advantages:
- Easy manufacturing.
- Usability as a pharmaceutical primary packaging.
- The contents of the vessel are protected from soiling.
- Easy and user-friendly handling.
- Height-independent horizontal sealing between cap and dispending-dropper.
- Height-independent horizontal sealing between dispending-dropper and vessel.
- Damage to the dispensing tip prevented.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### List of reference signs

- 1: vessel
- 2: dispensing dropper
- 3: dispensing tip
- 4: cap
- 5: fixation element
- 6: fixation element
- 7: sealing ring
- 8: lateral wall
- 9: basis body
- 10: lateral surface
- 11: longitudinal direction
- 12: distal end
- 13: end stop element
- 14: end stop element
- 15: undercut
- 16: undercut
- 17: lateral sealing surface
- 18: lateral sealing surface
- 19: tamper-evident ring
- 20: snap ring
- 21: snap ring
- 22: tamper-evident connecting element
- 23: centering ring
- 24: bottom
- 25: centering rib
- 26: corrugations
- 27: squeezing portion
- 28: distal opening
- 29: chamfer

## Claims

1. Device for dispensing a liquid, preferably an aqueous solution, comprising a vessel (1) for containing the liquid, a dispensing dropper (2) with a dispensing tip (3) and a cap (4), wherein the cap (4) and the vessel (1) comprise corresponding fixation (5, 6) elements,
**characterized in that** the cap (4) comprises a sealing ring (7) and that a lateral wall (8) of the sealing ring (7) is in engagement with a lateral surface (10) of the dispensing tip (3) when the vessel (1) is closed by the cap (4).

2. Device according to claim 1, **characterized in that** said fixation elements (5, 6) of the cap (4) and of the vessel (1) are corresponding threads.

3. Device according to claim 2, **characterized in that** said vessel (1) and said cap (4) comprise corresponding end stop elements (13, 14) and wherein the cap (4) can only be screwed onto the vessel (1) until the end stop elements (13, 14) engage with each other.

4. Device according to any one of claims 1 to 3, **characterized in that** said lateral wall (8) of the sealing ring (7) extends at least 2,0 mm, preferably at least 2,1 mm, from a basis body (9) of the cap (4), and/or wherein the lateral wall (8) of the sealing rings (7) extends less than 2,3 mm from a basis body (9) of the cap (4).

5. Device according to any one of claims 1 to 4, **characterized in that** said lateral surface (10) of the dispensing tip (3) extends at least 2,0 mm, preferably at least 2,3 mm, more preferably at least 1 mm, into a longitudinal (11) direction of the dispensing dropper (2), and/or wherein the lateral surface (10) of the dispensing tip (3) extends less than 2,5 mm into a longitudinal direction (11) of the dispensing dropper (2).

6. Device according to any one of claims 1 to 5, **characterized in that** said dispensing dropper (2) is form-fitted and/or force-fitted to the vessel (1),
and/or wherein a distal opening (28) of the dispensing tip (3) comprises a, preferably surrounding, chamfer (29).

7. Device according to any one of claims 1 to 6, **characterized in that** said dispensing dropper (2) and said vessel (1) each comprise an undercut (15, 16), preferably a snap ring (20, 21), such that the dispensing dropper (2) can be fixed to the vessel (1).

8. Device according to any one of claims 1 to 7, **characterized in that** a tamper-evident ring (19) is connected to the cap (4) and/or to the vessel (1), preferably form-fitted and/or force-fitted, and/or wherein a tamper-evident ring is integrally formed with the cap (4) and/or with the vessel (1).

9. Device according to any one of claims 2 to 8, **characterized in that** said vessel (1) comprises a centering element, preferably a centering ring (23), and wherein the centering element (23) is arranged between the thread and a bottom (24) of the vessel (1).

10. Device according to any one of claims 1 to 9, **characterized in that** said dispensing dropper (2) and said vessel (1) comprise corresponding lateral sealing surfaces (17, 18) and wherein lateral sealing surface (18) of the vessel (1) is in engagement with a corresponding lateral sealing surface (17) of the dispensing dropper (2).

11. Device according to any one of claims 1 to 10, **characterized in that** an opening torque for unscrewing the cap (4) from the vessel (1) is in the range of 25 N cm to 70 N·cm.

12. Device according to any one of claims 1 to 11, **characterized in that** said cap (4) comprises a high-density polyethylene, HDPE, and/or wherein said dispensing dropper (2) comprises a low density polyethylene, LDPE, and/or wherein said vessel (1) comprises a low density polyethylene, LDPE, and/or wherein a tamper-evident ring (19) comprises a low density polyethylene, LDPE.

13. Device according to any one of claims 1 to 12, **characterized in that** said vessel (1) comprises a squeezing portion (27) and wherein the vessel (1) is elastic against a force being applied to the squeezing portion (27) of up to 70 N, preferably of up to 60 N.

14. Device according to any one of claims 1 to 13, **characterized in that** a mounting force for connecting the dispensing dropper (2) to the vessel (1) is less than 100 N, preferably less than 90 N, and/or wherein the mounting force for connecting the dispensing dropper (2) to the vessel (1) is greater than 50 N,
and/or wherein a distal opening of the dispensing tip (3) comprises a diameter in the range from 0,3 mm to 2,5 mm, preferably from 0,8 mm to 2,3 mm.

15. Device according to any one of claims 1 to 14, **characterized in that** at least two, preferably at least three, more preferably four, centering ribs (25) are arranged at the distal tip (3) of the dispensing dropper (2), preferably wherein the centering ribs (25) are arranged equidistantly to each other when viewed in a circumferential direction.
